# EUROPEAN PATENT APPLICATION

(11) **EP 2 283 806 A1**
(43) Date of publication of application: **16.02.2011**
(21) Application number: 09167623.9
(22) Date of filing: 11.08.2009
(51) Int. Cl.: A61K 8/368, A61Q 5/00, A61Q 19/00

(54) **Carnosol for hair and skin care**

(71) Applicant: DSM IP Assets B.V., 6411 TE Heerlen (NL)
(72) Inventor: The designation of the inventor has not yet been filed
(74) Representative: Schwander, Kuno

(57) **Abstract**

This invention relates to the use of an oral composition comprising carnosol which enhances the appearance of hair and skin, and particularly has an anti-greying effect on the hair. It further relates methods of improving the appearance of hair and skin by oral administration of an effective amount of carnosol.

## Description

### FIELD OF THE INVENTION

This invention relates to the use of an oral composition comprising carnosol which enhances the appearance of hair and skin. It further relates methods of improving the appearance of hair or skin by oral administration of an effective amount of carnosol.

### BACKGROUND OF THE INVENTION

Greying of the hair is one external indication of the ageing process in humans and many other animals. In our modem society, people often go to great lengths to hide the greying, usually by dyeing hair, and in some cases, beards and moustaches. However, many consumers worry that the chemicals found in hair dye may not be completely safe to use for a prolonged period of time, and would prefer to use a more natural product.

Carnosol, which is an active ingredient in plant extracts, (such as rosemary and sage extracts) is well known as an antioxidant. It is widely used as an antioxidant for a variety of foodstuffs, including edible oils, such as fish oils.

Carnosol-containing compositions and carnosic acid-containing compositions for topical use on the hair and skin are also known. See, for example WO 2004/071473, describing a hair conditioning product containing carnosic acid which protects the colour of dyed hair. US 7,223,382 describes the use of DHA in combination with rosemary extract to prevent malodour formation in a self-tanning composition. Reuter et al, 2007 Planta Medica 73(11):1190-1191 describes a sage extract containing carnosol and carnosic acid which, when applied topically to the skin, reduced UV-induced erythema.

Carnosol-containing compositions have also been ingested. For example, US 7,270,835 teaches a nutraceutical containing a hops extract which is optionally combined with a rosemary extract used to modulate conditions arising from inflammation.

Further, Japanese patent application 2003-102122 teaches that carnosol, from rosemary plants, has anti-androgenic properties, and may be used for treatment of alopecia.

There is a need for natural products which can prevent premature greying of the hair.

### BRIEF DESCRIPTION OF THE INVENTION

It has been found, in accordance with this invention, that an oral composition comprising carnosol has the surprising benefit of improving the appearance of skin and hair. Thus one embodiment of this invention is an oral nutraceutical or food composition comprising carnosol in an amount effective to enhance the appearance of a mammal's hair and/or skin. Another embodiment of this invention is a method of enhancing the appearance of a mammal's hair or skin comprising administering an effective amount of an oral nutraceutical or food composition comprising carnosol.

It has also been found that mammals ingesting an effective amount of carnosol restored the natural dark colour of fur in old mice which naturally develop grey fur with age. Thus another aspect of this invention is a method for delaying the onset of greyness in hair and/or fur, and/or restoring natural hair colour in a mammal exhibiting greying related to ageing, comprising administering an effective amount of an oral formulation of carnosol.

Further, it has been found, according to this invention, that mammals ingesting carnosol have glossier hair and fur, compared to mammals receiving placebo. Thus another aspect of this invention is a method of enhancing hair or fur glossiness or shininess and general appearance comprising administering an oral composition containing carnosol.

It has also been found in accordance with this invention, that wounds which normally occur during the course of the animals' captive life healed more quickly if they had received carnosol. Thus, another aspect of this invention is a method of accelerating wound healing in a mammal with a skin wound comprising administering a wound-healing amount of a composition containing carnosol.

It has also been found in accordance with this invention, that mammals receiving an oral composition containing carnosol had an overall healthier appearance, were more "groomed" and had an overall better skin condition than those receiving placebo. Further investigation revealed that carnosol stimulates the production of the protein elastin, which is responsible for skin elasticity. Thus another aspect of this invention is a method of enhancing skin quality comprising administering an oral composition containing carnosol.

The compositions according to the invention are especially attractive, since many people, including animal owners and handlers, have a special interest in cosmetic treatments considered as "natural" with mild effects and without major side effects. As an orally available compound, carnosol has a further advantage in that it is easy to use, does not leave any residue on the hair or fur, and dosages are easier to control than in topical formulations.

In preferred embodiments of this invention, the carnosol is ingested regularly, for a chronic period of time, for example daily or several times during the day, for a minimum of two weeks, and preferably at least four weeks or until the beneficial effects are noticeable.

### BRIEF DESCRIPTION OF THE FIGURES

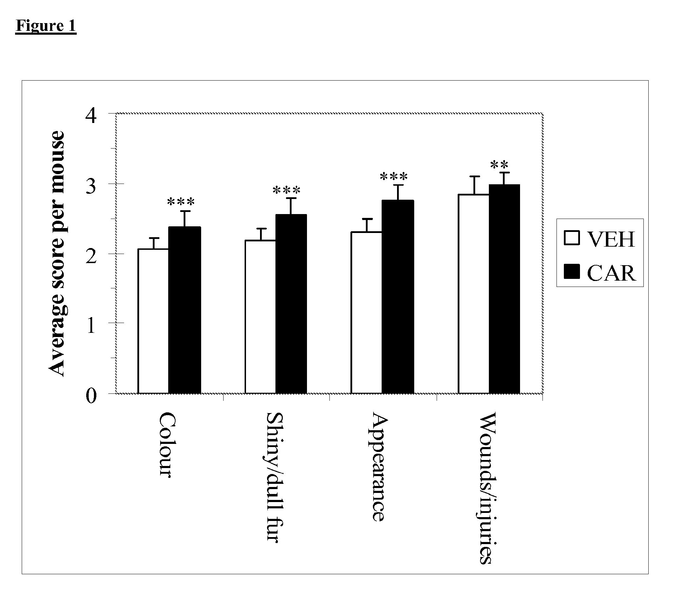

FIGURE 1 is a graph showing the effects of chronic carnosol treatment (200 mg/kg, p.o., 8 weeks) on fur and skin condition in middle-aged C57B1/6J mice. Carnosol (CAR) significantly improved both fur condition and the appearance of skin wounds, compared with vehicle-treated (VEH) control mice. Data are shown as mean ± S.D. (n = 13 per group) and statistical significance was calculated by unpaired t-tests, where **p < 0.01 and ***p < 0.001.

Carnosol, as used in this invention, may either be synthetically produced using known methods, or may be isolated from any number of plant species known to contain carnosol. Preferred plants include rosemary (*Rosmarinus ssp.)* and sage *(Salvia officinalis).* Extraction methods are known in the art. Plant extracts containing carnosol are widely commercially available, and make up a preferred embodiment of this invention.

The carnosol used in this invention may be in a pure form, or it may be admixed with other ingredients, such as those commonly found in commercially available plant extracts. References to dosages as used throughout this application refer to the amount of pure carnosol, but one of ordinary skill in the art can easily convert these dosages to their equivalent for non-pure forms, such as in an extract.

### DEFINITIONS

The term "nutraceutical composition" as used herein includes food products, foodstuffs, dietary supplements, nutritional supplements or a supplement composition for a food product or a foodstuff, including beverages (e.g., but not limited to, sports beverages, functional waters, juices, smoothies; instant drinks), soups, dairy products (e.g., but not limited to, single shot yogurt drinks), nutritional bars, and spreads.

As used herein, the term "food product" refers to any food or feed suitable for consumption by humans or animals.

The term "dietary supplement" refers to a small amount of a compound for supplementation of a human or animal diet packaged in single or multiple dose units. Dietary supplements do not generally provide significant amounts of calories but may contain other micronutrients (e.g. vitamins or minerals).

The term "nutritional supplement" refers to a composition comprising a dietary supplement in combination with a source of calories. In some embodiments, nutritional supplements are meal replacements or supplements (e.g. nutrient or energy bars or nutrient beverages or concentrates).

"Preventing" as used herein means not only halting the occurrence of a condition or event, but also delaying the onset of the condition or event, and lessening the severity of the condition or event when it does occur.

"Hair" means both hair of a human being or animal fur.

"Chronic administration" is meant to convey that administration of the active ingredient regularly occurs over an extended period of time, for example once or more per day for a period of at least about two weeks, preferably for at least one month, and more preferably for at least two months. Alternatively, the regular administration can be every two days, every three days, or once per week or twice per week.

"Extended period of time" means substantially daily for a period of time of at least about two weeks, preferably at least about a month, and even more preferably for at least about two months.

### Anti-Greying

More particularly, the present invention relates to the use of oral carnosol-containing compositions in hair care, particularly for the prevention of the greying of hair and/or for restoration and/or maintenance of the natural hair colour. It also relates to orally-administered hair care compositions, particularly for the prevention of the greying of hair and/or for restoration and/or maintenance of natural hair colour comprising carnosol and a carrier conventionally used for oral nutraceutical or food compositions. Furthermore, the invention relates to a method of preventing the greying of hair and/or restoring and/or maintaining the natural hair colour which comprises administering orally to a mammal in need of such treatment a composition comprising an effective amount of carnosol.

### Skin condition

In another embodiment the invention relates to a method of supporting healthy skin appearance and beauty, skin nourishment from inside the body via the blood stream (systemically), moisturising the skin, supporting the skin barrier function, fostering hydration, providing protection against UV-radiation and/or providing a general anti-aging effect, in particular promoting skin repair and/or regeneration upon healing of injuries and/or promoting the physiological renewal process and thus an optimal health, a natural radiance and glow and/or a beautiful appearance of the skin comprising the step of orally administering a composition containing an effective amount of carnosol.

### Veterinary Uses

In another aspect of this invention, the mammal is a non-human animal, such as a companion animal (dog, cat, ferret) or an animal which is used in the fur industry (minks, chinchillas or the like), or an animal which is shown in competition (such as dogs, cats, rabbits and other farm animals). Supplementing the animal's diet with the carnosol-containing compositions of this invention will enhance the appearance of the animals' fur. Thus another aspect of this invention is a veterinary nutraceutical or foodstuff containing a fur-enhancing amount of carnosol.

Another aspect of this invention is a supplement especially designed for a show animal which comprises carnosol. The animal should be fed this supplement daily for at least one month, and preferably for at least two months prior to the competition in order for its fur to be at its optimal condition. In preferred embodiments, the supplement is in the form of a treat or chew.

### Preferred Food Products

The food product may be a prepared and packaged food (e.g. mayonnaise, salad dressing, bread, or cheese food) or an animal feed (e.g. extruded and pelleted animal feed, coarse mixed feed, pet food composition, treats or chews).

Food products or foodstuffs are, for example, beverages such as non-alcoholic and alcoholic drinks as well as liquid preparations to be added to drinking water and liquid food. Non-alcoholic drinks are for instance: soft drinks, sport drinks, fruit juices (e.g. orange juice, apple juice and grapefruit juice); lemonades, teas, near-water drinks, milk and other dairy drinks (e.g. yoghurt drinks) and diet drinks. In another embodiment, food products or foodstuffs refer to solid or semi-solid foods comprising the composition according to the invention. These forms can include, but are not limited to, baked goods such as: cakes and cookies, puddings, dairy products, confections, snack foods, or frozen confections or novelties (e.g. ice cream, milk shakes), prepared frozen meals, candy, snack products (e.g. potato crisps/chips), liquid food such as soups, spreads, sauces, salad dressings, prepared meat products, cheese, yogurt and any other fat- or oil- containing foods, and food ingredients (e.g. wheat flour).

Animal feed, including pet food compositions, advantageously include food intended to supply necessary dietary requirements, as well as treats (e.g. dog biscuits) or other food supplements. The animal feed comprising the composition according to the invention may be in the form of a dry composition (e.g. kibble), semi-moist composition, wet composition, or any mixture thereof. Alternatively or additionally, the animal feed is a supplement, such as a gravy, drinking water, yogurt, powder, suspension, chew, treat (e.g. biscuits) or any other delivery form.

Dietary supplements of the present invention are formulated for oral delivery. The ingredients of the dietary supplement of this invention are contained in acceptable excipients and/or carriers for oral consumption. The actual form of the carrier, and thus, the dietary supplement itself, is not critical. The carrier may be a liquid, gel, gelcap, capsule, powder, solid tablet (coated or non-coated), tea, or the like. The dietary supplement is preferably in the form of a tablet or capsule and most preferably in the form of a hard (shell) gelatine capsule. Suitable excipients and/or carriers include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof). Preferred carriers include calcium carbonate, magnesium stearate, maltodextrin, and mixtures thereof. The various ingredients and the excipient and/or carrier are mixed and formed into the desired form using conventional techniques. The tablet or capsule of the present invention may be coated with an enteric coating that dissolves at a pH of about 6.0 to 7.0. A suitable enteric coating that dissolves in the small intestine but not in the stomach is cellulose acetate phthalate. Further details on techniques for formulation and administration may be found in the latest edition of Remington's Pharmaceutical Sciences (Maack Publishing Co., Easton, PA).

In other embodiments, the dietary supplement is provided as a powder or liquid suitable for adding by the consumer to a food or beverage. For example, in some embodiments, the dietary supplement can be administered to an individual in the form of a powder, for instance to be used by mixing into a beverage, or by stirring into a semi-solid food such as a pudding, topping, sauce, puree, cooked cereal, or salad dressing, for instance, or by otherwise adding to a food, e.g. enclosed in caps of food or beverage containers for release immediately before consumption. The dietary supplement may comprise one or more inert ingredients, especially if it is desirable to limit the number of calories added to the diet by the dietary supplement. For example, the dietary supplement of the present invention may also contain optional ingredients including, for example, herbs, vitamins, minerals, enhancers, colourants, sweeteners, flavourants, inert ingredients, and the like.

In some embodiments, the dietary supplements further comprise vitamins and minerals including, but not limited to, calcium phosphate or acetate, tribasic; potassium phosphate, dibasic; magnesium sulphate or oxide; salt (sodium chloride); potassium chloride or acetate; ascorbic acid; ferric orthophosphate; niacinamide; zinc sulphate or oxide; calcium pantothenate; copper gluconate; riboflavin; beta-carotene; pyridoxine hydrochloride; thiamine mononitrate; folic acid; biotin; chromium chloride or picolonate; potassium iodide; sodium selenate; sodium molybdate; phylloquinone; vitamin D3; cyanocobalamin; sodium selenite; copper sulphate; vitamin A; vitamin C; inositol; potassium iodide. Suitable dosages for vitamins and minerals may be obtained, for example, by consulting the U.S. RDA guidelines.

In other embodiments, the present invention provides nutritional supplements (e.g. energy bars or meal replacement bars or beverages) comprising the composition according to the invention. The nutritional supplement may serve as meal or snack replacement and generally provide nutrient calories. Preferably, the nutritional supplements provide carbohydrates, proteins, and fats in balanced amounts. The nutritional supplement can further comprise carbohydrate, simple, medium chain length, or polysaccharides, or a combination thereof. A simple sugar can be chosen for desirable organoleptic properties. Uncooked cornstarch is one example of a complex carbohydrate. If it is desired that it should maintain its high molecular weight structure, it should be included only in food formulations or portions thereof which are not cooked or heat processed since the heat will break down the complex carbohydrate into simple carbohydrates, wherein simple carbohydrates are mono- or disaccharides. The nutritional supplement contains, in one embodiment, combinations of sources of carbohydrate of three levels of chain length (simple, medium and complex; e.g. sucrose, maltodextrins, and uncooked cornstarch).

Sources of protein to be incorporated into the nutritional supplement of the invention can be any suitable protein utilised in nutritional formulations and can include whey protein, whey protein concentrate, whey powder, egg, soy flour, soy milk, soy protein, soy protein isolate, caseinate (e.g. sodium caseinate, sodium calcium caseinate, calcium caseinate, potassium caseinate), animal and vegetable protein and hydrolysates or mixtures thereof. When choosing a protein source, the biological value of the protein should be considered first, with the highest biological values being found in caseinate, whey, lactalbumin, egg albumin and whole egg proteins. In a preferred embodiment, the protein is a combination of whey protein concentrate and calcium caseinate. These proteins have high biological value; that is, they have a high proportion of the essential amino acids. See Modem Nutrition in Health and Disease, eighth edition, Lea & Febiger, publishers, 1986, especially Volume 1, pages 30-32.

The nutritional supplement can also contain other ingredients, such as one or a combination of other vitamins, minerals, antioxidants, fibre and other dietary supplements (e.g. protein, amino acids, choline, lecithin, omega-3 fatty acids). Selection of one or several of these ingredients is a matter of formulation, design, consumer preference and end-user. The amounts of these ingredients added to the dietary supplements of this invention are readily known to the skilled artisan. Guidance to such amounts can be provided by the U.S. RDA doses for children and adults. Further vitamins and minerals that can be added include, but are not limited to, calcium phosphate or acetate, tribasic; potassium phosphate, dibasic; magnesium sulphate or oxide; salt (sodium chloride); potassium chloride or acetate; ascorbic acid; ferric orthophosphate; niacinamide; zinc sulphate or oxide; calcium pantothenate; copper gluconate; riboflavin; beta-carotene; pyridoxine hydrochloride; thiamine mononitrate; folic acid; biotin; chromium chloride or picolonate; potassium iodide; sodium selenate; sodium molybdate; phylloquinone; vitamin D3; cyanocobalamin; sodium selenite; copper sulphate; vitamin A; vitamin C; Vitamin E, inositol; and potassium iodide. Moreover, a multi-vitamin and mineral supplement may be added to the nutraceutical compositions of the present invention to obtain an adequate amount of an essential nutrient, which is missing in some diets. The multi-vitamin and mineral supplement may also be useful for disease prevention and protection against nutritional losses and deficiencies due to lifestyle patterns.

The nutritional supplement can be provided in a variety of forms, and by a variety of production methods. In a preferred embodiment, to manufacture a food bar, the liquid ingredients are cooked; the dry ingredients are added with the liquid ingredients in a mixer and mixed until the dough phase is reached; the dough is put into an extruder, and extruded; the extruded dough is cut into appropriate lengths; and the product is cooled. The bars may contain other nutrients and fillers to enhance taste, in addition to the ingredients specifically listed herein.

It is understood by those of skill in the art that other ingredients can be added to those described herein, for example, fillers, emulsifiers, preservatives, etc. for the processing or manufacture of a nutritional supplement.

Additionally, flavours, colouring agents, spices, nuts and the like may be incorporated into the nutraceutical composition. Flavourings can be in the form of flavoured extracts, volatile oils, chocolate flavourings, peanut butter flavouring, cookie crumbs, crisp rice, vanilla or any commercially available flavouring. Examples of useful flavouring include, but are not limited to: pure anise extract, imitation banana extract, imitation cherry extract, chocolate extract, pure lemon extract, pure orange extract, pure peppermint extract, imitation pineapple extract, imitation rum extract, imitation strawberry extract, or pure vanilla extract; or volatile oils, such as balm oil, bay oil, bergamot oil, cedarwood oil, walnut oil, cherry oil, cinnamon oil, clove oil, or peppermint oil; peanut butter, chocolate flavouring, vanilla cookie crumb, butterscotch or toffee. In one embodiment, the dietary supplement contains cocoa or chocolate.

Emulsifiers may be added for stability of the nutraceutical compositions. Examples of suitable emulsifiers include, but are not limited to, lecithin (e.g. from egg or soy), and/or mono- and diglycerides. Other emulsifiers are readily apparent to the skilled artisan and selection of suitable emulsifier(s) will depend, in part, upon the formulation and final product. Preservatives may also be added to the nutritional supplement to extend product shelf life. Preferably, preservatives such as potassium sorbate, sodium sorbate, potassium benzoate, sodium benzoate or calcium disodium EDTA are used.

In addition to the carbohydrates described above, the nutraceutical composition can contain natural or artificial (preferably low calorie) sweeteners, e.g. saccharides, cyclamates, aspartamine, aspartame, acesulfame K, and/or sorbitol. Such artificial sweeteners can be desirable if the nutritional supplement is intended to be consumed by an overweight or obese individual, or an individual with type II diabetes who is prone to hyperglycaemia.

### Dosages

The preferred daily dosage of the subject composition as specified above may be administered in the form of one or more dosage units such as, e.g., a tablet. Most preferably the daily dosage of the subject composition is provided in the form of one dosage unit taken twice daily, for a total of two dosage units a day, or in the form of two dosage units taken twice daily, for a total of four dosage units a day. Compared to taking the total daily dose once a day, twice daily dosing of half the total daily dose in one or more dosage units per dose provides improved absorption and better maintenance of blood levels of the essential ingredients.

In a preferred embodiment, the carnosol-containing nutraceutical or food is eaten on a regular basis, i.e. at least daily for a sustained period of time (i.e. at least one week, preferably at least two weeks, and more preferably for at least three weeks, or at least one month) until the hair or skin enhancement is noted. After this time, the consumer may choose to lessen the dosage.

For human use, the recommended dosage of carnosol for the purposes of the present invention is in the range of from 0.001 mg per kg body weight to about 20 mg per kg body weight per day. More preferred is a daily dosage of from about 0.01 to about 10 mg per kg body weight, and especially preferred is a daily dosage of from about 0.05 to 5.0 mg per kg body weight. The amount of a plant material or plant extract containing carnosol can be calculated accordingly.

In solid dosage unit preparations for humans, carnosol is suitably present in an amount in the range of from about 0.1 mg to about 1000 mg, preferably in the range of from about 1 mg to about 500 mg per dosage unit.

In dietary compositions, especially in food and beverages for humans, carnosol is suitably present in an amount in the range of from about 0.0001 (1 mg/kg) to about 5 weight-% (50 g/kg), preferably from about 0.001 % (10 mg/kg) to about 1 weight-%, (10 g/kg) more preferably from about 0.01 (100 mg/kg) to about 0.5 weight-% (5 g/kg), based upon the total weight of the food or beverage.

In food and drinks, the range is from 10 to 30 mg per serving, i.e. 120 mg per kg food or drink.

For animals excluding humans a suitable daily dosage of carnosol may be within the range of from 0.001 mg per kg body weight to about 1000 mg per kg body weight per day. More preferred is a daily dosage in the range of from about 0.1 mg to about 500 mg per kg body weight, and especially preferred is a daily dosage in the range of from about 1 mg to 100 mg per kg body weight.

The following non-limiting Examples are presented to better illustrate the invention.

### Example 1

### Effects of carnosol treatment on fur and skin condition in wild-type mice

Middle-aged C57B1/6J mice (15 months of age) were administered carnosol (200 mg/kg, p.o.) or vehicle (water/corn oil, 1:1 mixture) daily, for eight weeks. At the end of the treatment period, mice were sacrificed and photographs were taken of each mouse. Eight participants, with experience of performing experimental studies using mice were asked to subjectively rate the condition of the fur and skin of each mouse, according to a three-point scale (see Table 1). The parameters rated for fur condition included fur colour, hair loss, "shininess" (glossiness) and general appearance (groomed/non-groomed); for skin condition, participants were requested to rate the appearance of wounds. The average score for each mouse, derived from the eight respondents' individual ratings, enabled subsequent comparison between carnosol-treated mice and vehicle-treated control mice.

Carnosol treatment significantly improved the fur condition of all mice, in comparison with control mice, as demonstrated by higher subjective rating scores for: (1) fur colour, i.e. fur appeared darker and younger-looking than that of control mice, (2) fur "shininess", i.e. hair appeared shinier/glossier than in control mice, and (3) fur appearance, i.e. fur was better groomed in carnosol-treated mice. In addition, carnosol-treated mice had lower incidences of wounds or conspicuous injuries, as demonstrated by higher subjective rating scores for this parameter compared with control mice (FIGURE 1).

**Table 1**

| | | |
|---|---|---|
| Fur and skin condition rating system. | | |
| **Fur colour:** | | |
| Grey and old | Not obvious | Dark and young |
| 1 □ | 2 □ | 3 □ |
| **Baldness/hair loss:** | | |
| Lots of hair loss | Some hair loss | Normal hair |
| 1 □ | 2 □ | 3 □ |
| **Shiny fur:** | | |
| Dull fur | Not obvious | Shiny and healthy |
| 1 □ | 2 □ | 3 □ |
| **Appearance of fur:** | | |
| Neglected and scrubby | Not obvious | Well-groomed |
| 1 □ | 2 □ | 3 □ |
| **Conspicuous wounds/injuries:** | | |
| Many | Few | None |
| 1 □ | 2 □ | 3 □ |

### Example 2

### Preparation of a soft gelatine capsule

A soft gelatine capsule is prepared comprising the following ingredients:

| **Ingredient** | **Amount per Capsule** |
|---|---|
| Carnosol | 200 mg |
| Lecithin | 50 mg |
| Soy bean oil | 250 mg |

### Example 3

### Preparation of an instant flavoured soft drink

| **Ingredient** | **Amount [g]** |
|---|---|
| Carnosol | 0.9 |
| Sucrose, fine powder | 922.7 |
| Ascorbic acid, fine powder | 2.0 |
| Citric acid anhydrous powder | 55.0 |
| Lemon flavour | 8.0 |
| Trisodium citrate anhydrous powder | 6.0 |
| Tricalciumphosphate | 5.0 |
| β-Carotene 1% CWS from DNP AG, Kaiseraugst, Switzerland | 0.4 |
| **Total amount** | **1000** |

All ingredients are blended and sieved through a 500 µm sieve. The resulting powder is put in an appropriate container and mixed in a tubular blender for at least 20 minutes. For preparing the drink, sufficient water is added to 125 g of the obtained mixed powder, to make up to one litre of beverage. The ready-to-drink soft drink contains ca. 30 mg carnosol per serving (250 ml).

### Example 4

### Preparation of a fortified non-baked cereal bar

| **Ingredient** | **Amount [g]** |
|---|---|
| Carnosol | 0.95 |
| Sugar | 114.55 |
| Water | 54.0 |
| Salt | 1.5 |
| Glucose syrup | 130.0 |
| Invert sugar syrup | 95.0 |
| Sorbitol Syrup | 35.0 |
| Palm kernel fat | 60.0 |
| Baking fat | 40.0 |
| Lecithin | 1.5 |
| Hardened palm-oil | 2.5 |
| Dried and cut apple | 63.0 |
| Cornflakes | 100.0 |
| Rice crispies | 120.0 |
| Wheat crispies | 90.0 |
| Roasted hazelnut | 40.0 |
| Skimmed milk powder | 45.0 |
| Apple flavour 74863-33 | 2.0 |
| Citric acid | 5.0 |
| **Total amount** | 1000 |

Carnosol is premixed with skimmed milk powder and placed in a planetary bowl mixer. Cornflakes and rice crispies are added and the total is mixed gently. Then the dried and cut apples are added. In a first cooking pot sugar, water and salt are mixed in the amounts given above (solution 1). In a second cooking pot glucose, invert- and sorbitol- syrup are mixed in the amounts given above (solution 2). A mixture of baking fat, palm kernel fat, lecithin and emulsifier is the fat phase. Solution 1 is heated to 110°C. Solution 2 is heated to 113°C and then cooled in a cold water bath. Afterwards solutions 1 and 2 are combined. The fat phase is melted at 75°C in a water bath. The fat phase is added to the combined mixture of solutions 1 and 2. Apple flavour and citric acid are added to the liquid sugar-fat mix. The liquid mass is added to the dry ingredients and mixed well in the planetary bowl mixer. The mass is put on a marble plate and rolled to the desired thickness. The mass is cooled down to room temperature and cut into pieces. The non-baked cereal bar contains ca. 25 mg carnosol per serving (30 g).

## Claims

1. A method of enhancing the overall appearance of a mammal's hair/fur and skin, comprising administering an oral nutraceutical or food composition comprising carnosol for a time sufficient and in an amount effective to enhance the overall appearance of a mammal's hair/fur and skin.

2. A method according to Claim 1 wherein the enhancement of the overall appearance is selected from the group consisting of: restoring hair/fur colour and delaying the onset of greyness in hair/fur.

3. A method according to Claim 1 wherein the enhancement comprises improved glossiness of hair or fur.

4. A method according to Claim 1 wherein the enhancement is selected from: improved skin condition or quality and faster wound healing.

5. A method according to any of Claims 1-4 wherein the mammal is human.

6. Use of carnosol in the manufacture of an oral nutraceutical or food composition which enhances the overall appearance of a mammal's hair and skin.

7. Use according to Claim 6 wherein the enhancement of the overall appearance is selected from the group consisting of: restoring hair/fur colour and delaying the onset of greyness in hair/fur.

8. Use according to Claim 6 wherein the enhancement comprises improved glossiness of hair or fur.

9. Use according to Claim 6 wherein the enhancement is selected from: improved skin condition or quality and faster wound healing.

10. Use according to any of Claims 6-9 wherein the mammal is human.
